# EUROPÄISCHE PATENTSCHRIFT

(11) **EP 3 171 925 B1**
(45) Veröffentlichungstag und Bekanntmachung des Hinweises auf die Patenterteilung: **13.11.2019**
(21) Anmeldenummer: 15738657.4
(22) Anmeldetag: 17.07.2015
(51) Int. Cl.: B65D 35/38, A61M 35/00, A61M 31/00

(54) **TUBE MIT APPLIKATIONSSPITZE**
TUBE WITH APPLICATION TIP
TUBE AVEC EMBOUT APPLICATEUR

(30) Priorität: 22.07.2014 EP 14177936
(43) Veröffentlichungstag der Anmeldung: 31.05.2017
(73) Patentinhaber: Bayer Animal Health GmbH, 51373 Leverkusen (DE)
(72) Erfinder: HABIG, Jörg, 51145 Köln (DE); HINXLAGE, Wilfried, 49413 Dinklage (DE)
(74) Vertreter: Tier 1 Patents
(86) Internationale Anmeldenummer: PCT/EP2015/066376
(87) Internationale Veröffentlichungsnummer: WO 2016/012355

(56) Entgegenhaltungen:
- EP-A1- 0 488 710
- WO-A2-2006/058138
- DE-U1-202013 103 549
- US-A- 3 949 871
- US-A1- 2013 108 352
- US-A1- 2013 345 673

## Beschreibung

Die Erfindung betrifft eine Tube mit Applikationsspitze für flüssiges oder pastöses Gut, insbesondere eine Wirkstoffformulierung, und ein Verfahren zur Herstellung und Befüllung der Tube mit Applikationsspitze. Solche Tuben enthalten bevorzugt eine Einmaldosis einer Wirkstoffformulierung z.B. eines Arzneimittels. Die Applikationsspitze dient der Einbringung des Wirkstoffs in Bereiche des menschlichen und tierischen Körpers, die schwierig zugänglich sind wie Körperöffnungen oder die Haut in Bereichen starker Behaarung.

Aus der US 2011/0160677 A1 ist ein Behälter für die Applikation einer Einmaldosis eines flüssigen Medikaments in die Mundhöhle eines Patienten bekannt. Der Behälter weist eine Kammer zur Aufnahme des flüssigen Medikaments auf und einen quetschbaren Bereich der benutzt wird, um das Medikament auszubringen. Der Behälter ist mit einem abdrehbaren Verschluss versiegelt, um ein vorzeitiges Ausbringen des Medikaments zu verhindern. Der Behälter weist weiterhin ein langgestrecktes Abgaberöhrchen von 1 bis 12 cm Länge auf, das in flüssiger Verbindung mit der Kammer steht. Zum Öffnen des Behälters greift der Nutzer den Behälter mit einer Hand. Mit Daumen und Zeigefinger der anderen Hand greift der Nutzer den Verschluss und übt eine Rotationskraft auf den Verschluss aus, so dass die Versiegelung an einer Sollbruchstelle bricht und der Verschluss von dem Behälter abgetrennt werden kann. Innerhalb des Behälters befindet sich außer der Einmaldosis an flüssigem Medikament inertes Gas, um zu verhindern, dass durch den Duck, den der Nutzer auf den Behälter ausübt, das flüssige Medikament aus dem Behälter austritt.

Ein Behälter ähnlicher Bauart und Handhabung ist in US 2007/0138215 A1 offenbart. Auch hier greift der Nutzer den Behälter mit einer Hand. Ein spezielles Mittel für das kraftschlüssige Festhalten und Drücken der Kammer mit der Flüssigkeit in Form von Furchen und Rillen ist außen auf der Kammer vorgesehen. Die Kammer wird vom Nutzer automatisch im Bereich der Furchen und Rillen ergriffen, wenn der Nutzer den abdrehbaren Verschluss entsiegeln und abdrehen möchte. Im Gegensatz zu dem Behälter in US 2011/0160677 A1 sind in dieser Patentanmeldung keine Maßnahmen offenbart, wie das Austreten von Flüssigkeit durch den Druck auf mit Flüssigkeit gefüllte Kammer beim Öffnen des Verschlusses verhindert werden kann.

Ein weiteres Behältnis für fließfähige Stoffe ist aus DE 44 20 594 A1 bekannt. Das Behältnis aus DE 44 20 594 A1 besteht aus einem Gehäuse aus Kunststoff, deren Kopf eine Ausgabeöffnung aufweist und einer sich an das offene Ende anschließenden offenen Kammer. Die Behältnisse dieser Art dienen der Aufnahme von Füllgütern. Durch manuelles Drücken kann der Inhalt aus dem Behältnis entnommen werden. Der Behälter ist mittels eines Verschlusses geschlossen, der längs einer Sollbruchstelle durch ein Kippen oder Drehen relativ zum Behälterkörper von diesem abgetrennt werden kann. Beim Öffnen des beschriebenen Behältnisses gemäß DE 44 20 594 A1 muss der Behältniskörper mit entsprechendem Druck festgehalten werden. Dabei ist es nachteilig, dass durch den Druck beim Öffnen des Verschlusses Flüssigkeit austreten kann.

DE 2013 103 549 U1 offenbart einen Behälter zum Dosieren eines fließfähigen Stoffes umfassend einen Aufnahmekörper welcher eine Umfangswand und einen Aufnahmekörperboden und eine an der dem Aufnahmekörperboden abgewandten Stirnseite des Aufnahmekörpers angeordnete Dosierspitze mit einer Austrittsöffnung zum Ausbringen des fließfähigen Stoffes. An der Umfangswand ist ein Hebel angeordnet, welcher sich entlang der Hauptachse des Aufnahmekörpers erstreckt und der mit der Umfangswand einen spitzen Winkel einschließt, so dass ein schlitzartiger Zwischenraum ausgebildet ist. Zum erstmaligen Gebrauch des Behälters muss ein Originalverschluss von der Dosierspitze getrennt werden, um einen Austragskanal der Dosierspitze freizugeben, wobei im Bereich zwischen der Dosierspitze und dem Originalverschluss eine Kerbe vorgesehen ist, welche ein Abtrennen des Originalverschlusses von der Dosierspitze erleichtert.

EP 0 488 710 A1 offenbart einen hermetisch dichten Behälter, der zur Abgabe eines Materials in einen Körperhohlraum geeignet ist. Der Behälter hat eine Applikationsspitze mit einer nahtlosen, hülsenförmigen, rotations-symmetrischen Wand. Vorzugsweise kann die Applikationsspitze mit einem Twist-Off-Verschluss versehen sein, der mit der Applikationsspitze durch eine zerbrechliche Verbindungsstelle verbunden ist, die die Abgabeöffnung der Applikationsspitze umgibt. Um die Ausübung einer Druckkraft auf den Kolben zu erleichtern, verfügt die Applikationsspitze auch über seitlich abstehende gegenüberliegende Fingerauflagen am ihrem proximalen Ende.

Der Erfindung liegt die Aufgabe zugrunde, eine Tube mit Applikationsspitze bereitzustellen, deren Verschluss geöffnet werden kann, ohne dass dabei der Tubenkörper festgehalten werden muss und so Druck auf den Tubenkörper ausgeübt wird. Gleichzeitig soll das Öffnen des Verschlusses an der Applikationsspitze keinen hohen Kraftaufwand erfordern und gleichzeitig ein unbeabsichtigtes Öffnen des Verschlusses vermieden werden.

Tube meint im Folgenden jede Art von quetschbarem Behälter. Erfindungsgemäß wird diese Aufgabe durch die Tube mit Applikationsspitze nach Anspruch 1 und das Herstellungsverfahren nach Anspruch 14 gelöst.

Die erfindungsgemäße Tube mit Applikationsspitze für flüssiges oder pastöses Gut, insbesondere Arzneimittel, weist einen Tubenkörper mit einem verschließbaren oder bereits verschlossenen Ende und einem Austrittsende auf. Das Austrittsende der Tube weist einen Tubenhals mit einer Austrittsöffnung auf. Die erfindungsgemäße Tube weist weiterhin eine in Längsrichtung der Tube gestreckte, mit dem Austrittsende des Tubenkörpers verbundene, Applikationsspitze mit einem inneren Kanal, der eine Eintrittsöffnung und eine Applikationsöffnung verbindet, auf. An die Applikationsöffnung der Applikationsspitze ist ein Versschluss angeformt, der die Applikationsöffnung verschließt. Die Applikationsspitze weist ein Griffmittel als Haltepunkt beim Öffnen der Tube auf, das sich über einen Teilbereich der Länge der Applikationsspitze erstreckt und als Verbreiterung in einer Dimension des äußeren Querschnitts der Applikationsspitze ausgeführt ist. Erfindungsgemäß ist der Verschluss mit zwei Verlängerungsstücken, die in Richtung Tubenkörper über die Applikationsöffnung hinausragen, über zwei Sollbruchstellen am Ende der Verlängerungsstücke auf gegenüberliegenden Seiten mit der Wand der Applikationsspitze verbunden.

In einer Ausführungsform der Erfindung ist die Applikationsspitze an den Tubenhals angeformt, so dass die Austrittsöffnung des Tubenkörpers und die Eintrittsöffnung inneren Kanals der Applikationsspitze flüssigkeitsdicht miteinander verbunden sind.

Das Griffmittel dient dazu beim Öffnen der Tube einen Haltepunkt zu haben. Bevorzugt ist der Bereich des Griffmittels etwas dicker vom Material her ausgeprägt, zum Beispiel durch eine entsprechende Versteifung vorhanden, die bei Druck nicht nachgibt.

Die Applikationsspitze weist das Griffmittel über ihre gesamte Länge oder über einen Teilbereich ihrer Länge auf. Das Griffmittel kann als Verbreiterung (in einer Dimension) des äußeren und gegebenenfalls auch des inneren Querschnitts der Applikationsspitze gegenüber dem äußeren bzw. inneren Querschnitt in anderen Teilbereichen der Applikationsspitze ausgeführt sein. Die Applikationsspitze weist das Griffmittel bevorzugt im Bereich der Eintrittsöffnung auf. Das Griffmittel kann als Griffmulde, bevorzugt mit Furchen und Rippen ausgestaltet sein.

Die Applikationsspitze kann durch Verschweißen formschlüssig an den Tubenhals angeformt sein.

Tubenkörper und Applikationsspitze sind aus Kunststoff hergestellt, bevorzugt aus Polypropylene (PP). Sie können aber in anderen Ausführungsformen auch aus Polyethylen (PE), Polyethylenterephthalat (PET), Polyvinylchlorid (PVC) oder Polyamid (PA) hergestellt sein. In einer Ausführungsform der Erfindung ist die Wanddicke der Applikationsspitze größer als die Wanddicke des Tubenkörpers. Die Wanddicke des Tubenkörpers kann im Bereich von 0,2 mm bis 0,6 mm liegen und die Wanddicke der Applikationsspitze im Bereich von 0,3 bis 1,2 mm. Im Bereich des Griffmittels kann die Wanddicke der Applikationsspitze größer sein als in den übrigen Teilbereichen der Applikationsspitze. Die Wanddicke im Bereich des Griffmittels kann größer sein als die Wanddicke der übrigen Teilbereiche der Applikationsspitze. Sie kann im Bereich von 0,4 bis 1,4 mm liegen.

Die Länge der Applikationsspitze ohne Verschluss sollte mindestens 20 % der Länge des Tubenkörpers betragen, bevorzugt mindestens 50% und besonders bevorzugt größer oder gleich der Länge des Tubenkörpers sein. Bevorzugt liegt die Länge der Applikationsspitze ohne Verschluss im Bereich von 15 mm bis 70 mm und die Länge des Tubenkörpers im Bereich von 15 mm bis 70 mm.

In einer weiteren Ausführungsform der Erfindung ist das Material der Applikationsspitze transparent. Das Material des Tubenkörpers ist bevorzugt nicht transparent.

Das verschließbare oder bereits verschlossene Ende des Tubenkörpers kann durch Quersiegelung verschlossen sein bzw. werden.

Der Verschluss kann über eine oder mehrere Sollbruchstellen mit der Applikationsspitze verbunden sein. Er ist bevorzugt abknickbar oder abdrehbar von der Applikationsspitze.

Die Tube mit Applikationsspitze enthält in einer Ausführungsform eine Einzeldosis Arzneimittel-Formulierung.

Der innere Kanal in der Applikationsspitze hat bevorzugt einen Durchmesser im Bereich von 0,5 bis 0,8 mm.

Weiterer Gegenstand der Erfindung ist ein Verfahren zur Herstellung einer befüllten Tube mit Applikationsspitze enthaltend die Schritte
a. Formung eines Tubenkörpers mit einem offenen Ende und einem Austrittsende, das einen Tubenhals mit einer Austrittsöffnung aufweist,
b. Formung einer Applikationsspitze mit einem inneren Kanal, der eine Eintrittsöffnung und eine Applikationsöffnung verbindet und einem Verschluss, der die Applikationsspitze verschließt,
c. Anformen der Applikationsspitze mit dem Bereich um ihre Eintrittsöffnung an den Tubenhals,
d. Befüllen des Tubenkörpers mit einem flüssigen oder pastösen Gut über das offene Ende,
e. Quersiegelung des offenen Endes;
wobei die Applikationsspitze ein Griffmittel als Haltepunkt beim Öffnen der Tube aufweist, das sich über einen Teilbereich der Länge der Applikationsspitze erstreckt und als Verbreiterung in einer Dimension des äußeren Querschnitts der Applikationsspitze ausgeführt ist, und dass der Verschluss mit zwei Verlängerungsstücken, die in Richtung Tubenkörper über die Applikationsöffnung hinausragen, über zwei Sollbruchstellen am Ende der Verlängerungsstücke auf gegenüberliegenden Seiten mit der Wand der Applikationsspitze verbunden ist.

### Figuren und Beispiele

Ein Ausführungsbeispiel der Erfindung soll unter Bezugnahme auf die Figuren der Zeichnung erläutert werden. Es zeigen:
- Fig. 1: zeigt eine Tube mit Applikationsspitze in perspektivischer Ansicht
- Fig. 2: zeigt eine Tube mit Applikationsspitze in Seitenansicht
- Fig. 3: zeigt eine Tube mit Applikationsspitze in Vorderansicht
- Fig. 4: zeigt eine Tube mit Applikationsspitze von unten

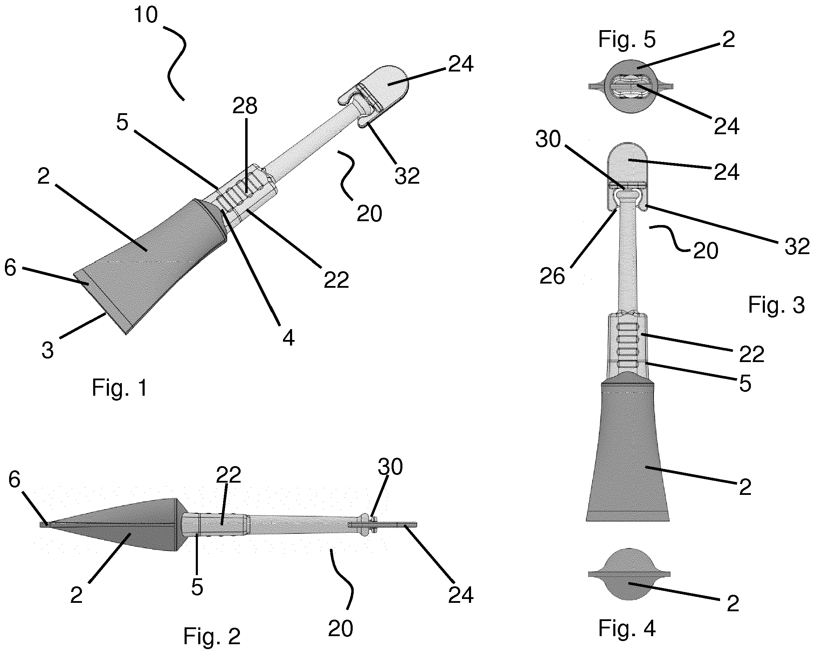

- Fig. 4: zeigt eine Tube mit Applikationsspitze von oben

Die in den Fig. 1-5 abgebildete Tube mit Applikationsspitze 10 besteht aus einem Tubenkörper 2 und einer Applikationsspitze 20.

Beim dargestellten Ausführungsbeispiel ist der Tubenkörper 2 an seinem Ende 3 durch eine Quersiegelnaht 6 verschlossen. Um eine einfache Öffnung und Applikation zu ermöglichen, ist der Tubenkörper 2 mit einer langen Applikationsspitze 20 mit einem leicht zu öffnenden Verschluss 24, der die Applikationsöffnung 30 verschließt, versehen. Der Verschluss 24 kann abgedreht oder abgeknickt werden. Der Verschluss ist mit zwei Verlängerungsstücken 32, die in Richtung Tubenkörper über die Applikationsöffnung 30 hinausragen und über zwei Sollbruchstellen 26 am Ende der Verlängerungsstücke auf gegenüberliegenden Seiten mit der Wand der Applikationsspitze 20 verbunden. Die lange Applikationsspitze 20 dient dazu, dass auch bei schwer zugänglichen Bereichen z.B. im Tierfell oder in Körperöffnungen eine Applikation des Tubeninhalts problemlos möglich ist. Die Applikationsspitze 20 kann transparent gestaltet sein, so dass man einen Austritt des Füllgutes sehen kann. Das Austrittsende 4 der Tube mit Applikationsspitze 10 weist einen Tubenhals 5 mit einer hier nicht sichtbaren Austrittsöffnung auf. Der durch die transparente Applikationsspitze 20 nur schwach sichtbare Tubenhals 5 ist durch eine Linie angedeutet. Die Applikationsspitze 20 ist formschlüssig an den Tubenhals 5 angeformt. Die Applikationsspitze 20 weist im Bereich der Eintrittsöffnung mit dem sie auch an den Tubenhals 5 angeformt ist, eine Griffmulde 22 mit Querrippen 28 auf, wobei die Griffmulde 22 über den Tubenhals 5 hinausgeht.

## Patentansprüche

1. Tube (10) mit Applikationsspitze (20) für flüssiges oder pastöses Gut, insbesondere eine Wirkstoffformulierung, aufweisend
a. einen Tubenkörper (2) mit einem verschließbaren oder bereits verschlossenen Ende (3) und einem Austrittsende (4), das einen Tubenhals (5) mit einer Austrittsöffnung ausweist,
b. eine in Längsrichtung der Tube (10) gestreckte, mit dem Austrittsende (4) des Tubenkörpers (2) verbundene, Applikationsspitze (20) mit einem inneren Kanal, der eine Eintrittsöffnung und eine Applikationsöffnung (30) verbindet und
c. einen Versschluss (24), der an die Applikationsöffnung (30) der Applikationsspitze (20) angeformt ist und die Applikationsöffnung (30) verschließt,
**dadurch gekennzeichnet, dass** die Applikationsspitze (20) ein Griffmittel (22,28) als Haltepunkt beim Öffnen der Tube aufweist, das sich über einen Teilbereich der Länge der Applikationsspitze (20) erstreckt und als Verbreiterung in einer Dimension des äußeren Querschnitts der Applikationsspitze (20) ausgeführt ist, und
dass der Verschluss (24) mit zwei Verlängerungsstücken (32), die in Richtung Tubenkörper (2) über die Applikationsöffnung (30) hinausragen, über zwei Sollbruchstellen (26) am Ende der Verlängerungsstücke (32) auf gegenüberliegenden Seiten mit der Wand der Applikationsspitze (20) verbunden ist.

2. Tube (10) mit Applikationsspitze (20) nach Anspruch 1, **dadurch gekennzeichnet, dass** die Applikationsspitze (20) an den Tubenhals (5) angeformt ist, so dass das Austrittsende (4) des Tubenkörpers (2) und die Eintrittsöffnung des inneren Kanals der Applikationsspitze (20) flüssigkeitsdicht miteinander verbunden sind.

3. Tube (10) mit Applikationsspitze (20) nach einem der Ansprüche 1, 2, **dadurch gekennzeichnet, dass** das Griffmittel (22,28) auch als Verbreiterung in einer Dimension des Querschnitts des inneren Kanals der Applikationsspitze (20) gegenüber dem Querschnitts des inneren Kanals in anderen Teilbereichen der Applikationsspitze (20) ausgeführt ist, wobei die Dimension dieselbe ist wie bei der Verbreiterung des äußeren Querschnitts.

4. Tube (10) mit Applikationsspitze (20) nach einem oder mehreren der vorhergehenden Ansprüche, **dadurch gekennzeichnet, dass** die Applikationsspitze (20) das Griffmittel (22,28) im Bereich der Eintrittsöffnung aufweist.

5. Tube (10) mit Applikationsspitze (20) nach einem oder mehreren der vorhergehenden Ansprüche, **dadurch gekennzeichnet, dass** das Griffmittel als Griffmulde (22) bevorzugt mit Furchen und Rippen (28) ausgestaltet ist.

6. Tube (10) mit Applikationsspitze (20) nach einem oder mehreren der vorhergehenden Ansprüche, **dadurch gekennzeichnet, dass** die Applikationsspitze (20) durch Verschweißen an den Tubenhals (5) formschlüssig angeformt ist.

7. Tube (10) mit Applikationsspitze (20) nach einem oder mehreren der vorhergehenden Ansprüche, **dadurch gekennzeichnet, dass** Tubenkörper (2) und Applikationsspitze (20) jeweils aus Polypropylene (PP), Polyethylen (PE), Polyethylenterephthalat (PET), Polyvinylchlorid (PVC) oder Polyamid (PA) hergestellt sind.

8. Tube (10) mit Applikationsspitze (20) nach einem oder mehreren der vorhergehenden Ansprüche, **dadurch gekennzeichnet, dass** die Wanddicke der Applikationsspitze (20) größer ist als die Wanddicke des Tubenkörpers (2), und dass die Wanddicke des Tubenkörpers (2) bevorzugt im Bereich von 0,2 mm bis 0,6 mm liegt, und die Wanddicke der Applikationsspitze (20) bevorzugt im Bereich von 0,3 mm bis 1,2 mm liegt.

9. Tube (10) mit Applikationsspitze (20) nach Anspruch 5, **dadurch gekennzeichnet, dass** die Wanddicke der Applikationsspitze (20) im Bereich der Griffmulde (22) höher ist als in den übrigen Teilbereichen der Applikationsspitze (20) und im Bereich von 0,4 mm bis 1,4 mm liegt.

10. Tube (10) mit Applikationsspitze (20) nach einem oder mehreren der vorhergehenden Ansprüche, **dadurch gekennzeichnet, dass** die Länge der Applikationsspitze (20) ohne Verschluss (24) mindestens 20 % der Länge des Tubenkörpers (2) beträgt, bevorzugt mindestens 50% und besonders bevorzugt größer oder gleich der Länge des Tubenkörpers (2) ist und bevorzugt im Bereich von 15 mm bis 70 mm liegt.

11. Tube (10) mit Applikationsspitze (20) nach einem oder mehreren der vorhergehenden Ansprüche, **dadurch gekennzeichnet, dass** das Material der Applikationsspitze (20) transparent ist.

12. Tube (10) mit Applikationsspitze (20) nach einem oder mehreren der vorhergehenden Ansprüche, **dadurch gekennzeichnet, dass** der Verschluss (24) über eine oder mehrere Sollbruchstellen (26) mit der Applikationsspitze (20) verbunden ist und bevorzugt abknickbar oder abdrehbar ist.

13. Tube (10) mit Applikationsspitze (20) nach einem oder mehreren der vorhergehenden Ansprüche, **dadurch gekennzeichnet, dass** der innere Kanal einen Durchmesser im Bereich von 0,5 mm bis 0,8 mm hat.

14. Verfahren zur Herstellung einer befüllten Tube (10) mit Applikationsspitze (20) enthaltend die Schritte
a. Formung eines Tubenkörpers (2) mit einem offenen Ende (3) und einem Austrittsende (4), das einen Tubenhals (5) mit einer Austrittsöffnung aufweist,
b. Formung einer Applikationsspitze (20) mit einem inneren Kanal, der eine Eintrittsöffnung und eine Applikationsöffnung (30) verbindet und einem Verschluss (24), der die Applikationsspitze (20) verschließt,
c. Anformen der Applikationsspitze (20) mit dem Bereich um ihre Eintrittsöffnung an den Tubenhals (5),
d. Befüllen des Tubenkörpers (2) mit einem flüssigen oder pastösen Gut über das offene Ende,
e. Quersiegelung des offenen Endes (3);
**dadurch gekennzeichnet, dass**
die Applikationsspitze (20) ein Griffmittel (22,28) als Haltepunkt beim Öffnen der Tube aufweist, das sich über einen Teilbereich der Länge der Applikationsspitze (20) erstreckt und als Verbreiterung in einer Dimension des äußeren Querschnitts der Applikationsspitze (20) ausgeführt ist, und
dass der Verschluss (24) mit zwei Verlängerungsstücken (32), die in Richtung Tubenkörper (2) über die Applikationsöffnung (30) hinausragen, über zwei Sollbruchstellen (26) am Ende der Verlängerungsstücke (32) auf gegenüberliegenden Seiten mit der Wand der Applikationsspitze (20) verbunden ist.

## Claims

1. Tube (10) with an application tip (20) for liquid or pasty material, in particular an active agent formulation, having
a. a tube body (2) with an end (3) which can be closed or has already been closed and with an outlet end (4) which has a tube neck (5) with an outlet opening,
b. an application tip (20) which is elongated in the longitudinal direction of the tube (10), is connected to the outlet end (4) of the tube body (2) and has an inner channel which connects an inlet opening and an application opening (30), and
c. a closure (24) which is moulded onto the application opening (30) of the application tip (20) and closes the application opening (30),
**characterized in that** the application tip (20) has a gripping means (22, 28) as holding point when opening the tube, which gripping means extends over a part region of the length of the application tip (20) and is configured as a widened portion in one dimension of the outer cross section of the application tip (20), and
**in that** the closure (24) is connected to the wall of the application tip (20) on opposite sides by way of two extension pieces (32) which protrude beyond the application opening (30) in the direction of the tube body (2), via two predetermined break points (26) at the end of the extension pieces (32).

2. Tube (10) with an application tip (20) according to Claim 1, **characterized in that** the application tip (20) is moulded onto the tube neck (5), with the result that the outlet end (4) of the tube body (2) and the inlet opening of the inner channel of the application tip (20) are connected to one another in a liquid-tight manner.

3. Tube (10) with an application tip (20) according to either of Claims 1 and 2, **characterized in that** the gripping means (22, 28) is also configured as a widened portion in one dimension of the cross section of the inner channel of the application tip (20) with respect to the cross section of the inner channel in other part regions of the application tip (20), wherein the dimension is the same as in the case of the widened portion of the outer cross section.

4. Tube (10) with an application tip (20) according to one or more of the preceding claims, **characterized in that** the application tip (20) has the gripping means (22, 28) in the region of the inlet opening.

5. Tube (10) with an application tip (20) according to one or more of the preceding claims, **characterized in that** the gripping means is configured as a recessed grip (22), preferably with grooves and ribs (28).

6. Tube (10) with an application tip (20) according to one or more of the preceding claims, **characterized in that** the application tip (20) is moulded onto the tube neck (5) in a positively locking manner by way of welding.

7. Tube (10) with an application tip (20) according to one or more of the preceding claims, **characterized in that** the tube body (2) and the application tip (20) are produced in each case from polypropylenes (PP), polyethylene (PE), polyethylene terephthalate (PET), polyvinyl chloride (PVC) or polyamide (PA).

8. Tube (10) with an application tip (20) according to one or more of the preceding claims, **characterized in that** the wall thickness of the application tip (20) is greater than the wall thickness of the tube body (2), and **in that** the wall thickness of the tube body (2) lies preferably in the range from 0.2 mm to 0.6 mm, and the wall thickness of the application tip (20) lies preferably in the range from 0.3 mm to 1.2 mm.

9. Tube (10) with an application tip (20) according to Claim 5, **characterized in that** the wall thickness of the application tip (20) in the region of the recessed grip (22) is higher than in the remaining part regions of the application tip (20) and lies in the range from 0.4 mm to 1.4 mm.

10. Tube (10) with an application tip (20) according to one or more of the preceding claims, **characterized in that** the length of the application tip (20) without closure (24) is at least 20% of the length of the tube body (2), preferably at least 50% and particularly preferably greater than or equal to the length of the tube body (2) and preferably in the range from 15 mm to 70 mm.

11. Tube (10) with an application tip (20) according to one or more of the preceding claims, **characterized in that** the material of the application tip (20) is transparent.

12. Tube (10) with an application tip (20) according to one or more of the preceding claims, **characterized in that** the closure (24) is connected to the application tip (20) via one or more predetermined break points (26) and preferably can be broken off by bending or by twisting.

13. Tube (10) with an application tip (20) according to one or more of the preceding claims, **characterized in that** the inner channel has a diameter in the range from 0.5 mm to 0.8 mm.

14. Method for producing a filled tube (10) with an application tip (20), including the steps
a. forming of a tube body (2) with an open end (3) and with an outlet end (4) which has a tube neck (5) with an outlet opening,
b. forming of an application tip (20) with an inner channel which connects an inlet opening and an application opening (30), and a closure (24) which closes the application tip (20),
c. moulding of the application tip (20) by way of the region around its inlet opening onto the tube neck (5),
d. filling of the tube body (2) with a liquid or pasty material via the open end,
e. transverse sealing of the open end (3);
**characterized in that**
the application tip (20) has a gripping means (22, 28) as holding point when opening the tube, which gripping means extends over a part region of the length of the application tip (20) and is configured as a widened portion in one dimension of the outer cross section of the application tip (20), and
**in that** the closure (24) is connected to the wall of the application tip (20) on opposite sides by way of two extension pieces (32) which protrude beyond the application opening (30) in the direction of the tube body (2), via two predetermined break points (26) at the end of the extension pieces (32).

## Revendications

1. Tube (10) comprenant un embout applicateur (20) pour produit liquide ou pâteux, en particulier une formulation de principe actif, comprenant
a. un corps de tube (2) doté d'une extrémité (3) pouvant être fermée ou déjà fermée et d'une extrémité de sortie (4) qui comprend un col de tube (5) doté d'une ouverture de sortie,
b. un embout applicateur (20) allongé dans la direction longitudinale du tube (10), relié à l'extrémité de sortie (4) du corps de tube (2) et doté d'un canal intérieur qui relie une ouverture d'entrée et une ouverture d'application (30) et
c. une fermeture (24) qui est moulée sur l'ouverture d'application (30) de l'embout applicateur (20) et ferme l'ouverture d'application (30),
**caractérisé en ce que** l'embout applicateur (20) comprend un moyen de préhension (22, 28) en tant que point de retenue lors de l'ouverture du tube, lequel moyen de préhension s'étend sur une région partielle de la longueur de l'embout applicateur (20) et est réalisé en tant qu'élargissement dans une dimension de la section transversale extérieure de l'embout applicateur (20) et
**en ce que** la fermeture (24) est reliée à la paroi de l'embout applicateur (20) par deux pièces de prolongement (32) qui font saillie au-delà de l'ouverture d'application (30) dans la direction du corps de tube (2), et par le biais de deux points de rupture imposée (26) à l'extrémité des pièces de prolongement (32) sur des côtés opposés.

2. Tube (10) comprenant un embout applicateur (20) selon la revendication 1, **caractérisé en ce que** l'embout applicateur (20) est moulé sur le col de tube (5), de telle sorte que l'extrémité de sortie (4) du corps de tube (2) et l'ouverture d'entrée du canal intérieur de l'embout applicateur (20) soient reliées l'une à l'autre de manière étanche aux liquides.

3. Tube (10) comprenant un embout applicateur (20) selon la revendication 1 ou 2, **caractérisé en ce que** le moyen de préhension (22, 28) est également réalisé en tant qu'élargissement dans une dimension de la section transversale du canal intérieur de l'embout applicateur (20) par rapport à la section transversale du canal intérieur dans d'autres régions partielles de l'embout applicateur (20), la dimension de celui-ci étant identique à celle de l'élargissement de la section transversale extérieure.

4. Tube (10) comprenant un embout applicateur (20) selon l'une ou plusieurs des revendications précédentes, **caractérisé en ce que** l'embout applicateur (20) comprend le moyen de préhension (22, 28) dans la région de l'ouverture d'entrée.

5. Tube (10) comprenant un embout applicateur (20) selon l'une ou plusieurs des revendications précédentes, **caractérisé en ce que** le moyen de préhension est configuré en tant que creux préhension (22) de préférence doté de rainures et de nervures (28).

6. Tube (10) comprenant un embout applicateur (20) selon l'une ou plusieurs des revendications précédentes, **caractérisé en ce que** l'embout applicateur (20) est moulé avec complémentarité de forme sur le col de tube (5) par soudage.

7. Tube (10) comprenant un embout applicateur (20) selon l'une ou plusieurs des revendications précédentes, **caractérisé en ce que** le corps de tube (2) et l'embout applicateur (20) sont produits respectivement à partir de polypropylène (PP), de polyéthylène (PE), de polyéthylènetéréphtalate (PET), de polychlorure de vinyle (PVC) ou de polyamide (PA).

8. Tube (10) comprenant un embout applicateur (20) selon l'une ou plusieurs des revendications précédentes, **caractérisé en ce que** l'épaisseur de paroi de l'embout applicateur (20) est supérieure à l'épaisseur de paroi du corps de tube (2), et **en ce que** l'épaisseur de paroi du corps de tube (2) est de préférence comprise dans la plage de 0,2 mm à 0,6 mm, et l'épaisseur de paroi de l'embout applicateur (20) est de préférence comprise dans la plage de 0,3 mm à 1,2 mm.

9. Tube (10) comprenant un embout applicateur (20) selon la revendication 5, **caractérisé en ce que** l'épaisseur de paroi de l'embout applicateur (20) dans la région du creux de préhension (22) est supérieure à celle dans les régions partielles restantes de l'embout applicateur (20) et est comprise dans la plage de 0,4 mm à 1,4 mm.

10. Tube (10) comprenant un embout applicateur (20) selon l'une ou plusieurs des revendications précédentes, **caractérisé en ce que** la longueur de l'embout applicateur (20) sans fermeture (24) est égale à au moins 20 % de la longueur du corps de tube (2), est de préférence d'au moins 50% et de manière particulièrement préférée supérieure ou égale à la longueur du corps de tube (2), et est de préférence comprise dans la plage de 15 mm à 70 mm.

11. Tube (10) comprenant un embout applicateur (20) selon l'une ou plusieurs des revendications précédentes, **caractérisé en ce que** le matériau de l'embout applicateur (20) est transparent.

12. Tube (10) comprenant un embout applicateur (20) selon l'une ou plusieurs des revendications précédentes, **caractérisé en ce que** la fermeture (24) est reliée à l'embout applicateur (20) par un ou plusieurs points de rupture imposée (26) et peut être de préférence rompue par flexion ou par torsion.

13. Tube (10) comprenant un embout applicateur (20) selon l'une ou plusieurs des revendications précédentes, **caractérisé en ce que** le canal intérieur présente un diamètre dans la plage de 0,5 mm à 0,8 mm.

14. Procédé de fabrication d'un tube rempli (10) comprenant un embout applicateur (20), le procédé comprenant les étapes de
a. formage d'un corps de tube (2) doté d'une extrémité ouverte (3) et d'une extrémité de sortie (4) qui comprend un col de tube (5) doté d'une ouverture de sortie,
b. formage d'un embout applicateur (20) doté d'un canal intérieur qui relie une ouverture d'entrée et une ouverture d'application (30) et d'une fermeture (24) qui ferme l'embout applicateur (20),
c. moulage de l'embout applicateur (20) par la région autour de son ouverture d'entrée sur le col de tube (5),
d. remplissage du corps de tube (2) avec un produit liquide ou pâteux par le biais de l'extrémité ouverte,
e. scellage transversal de l'extrémité ouverte (3) ;
**caractérisé en ce que**
l'embout applicateur (20) comprend un moyen de préhension (22, 28) en tant que point de retenue lors de l'ouverture du tube, lequel moyen de préhension s'étend sur une région partielle de la longueur de l'embout applicateur (20) et est réalisé en tant qu'élargissement dans une dimension de la section transversale extérieure de l'embout applicateur (20) et
**en ce que** la fermeture (24) est reliée à la paroi de l'embout applicateur (20) par deux pièces de prolongement (32) qui font saillie au-delà de l'ouverture d'application (30) dans la direction du corps de tube (2), et par le biais de deux points de rupture imposée (26) à l'extrémité des pièces de prolongement (32) sur des côtés opposés.
